# EUROPEAN PATENT SPECIFICATION

(11) **EP 0 744 185 B1**
(45) Date of publication and mention of the grant of the patent: **30.07.2003**
(21) Application number: 96201437.9
(22) Date of filing: 23.05.1996
(51) Int. Cl.: A61M 25/01, A61M 16/00, A61J 15/00

(54) **Catheter**
Katheter
Cathéter

(30) Priority: 23.05.1995 NL 1000424
(43) Date of publication of application: 27.11.1996
(73) Proprietor: N.V. Nutricia, 2700 MA Zoetermeer (NL)
(72) Inventor: Geldof, Han, 2986 TE Ridderkerk (NL)
(74) Representative: Hoijtink, Reinoud

(56) References cited:
- WO-A-93/03777
- WO-A-93/21984
- DE-A- 2 928 635
- DE-C- 3 323 482
- US-A- 3 894 540
- US-A- 4 819 619

## Description

The present invention relates to an assembly of a catheter and a flexible sheath, such a catheter, is known from DE-A-3323482.

This catheter is intended for insertion into the bronchial passages. The catheter is fixed by means of a vulnerable part in the catheter, a so-called balloon, which is inflated and places itself against the wall of the trachea. The flexible sheet is arranged in order to protect this balloon from damage during insertion. When the sheath is withdrawn it is pulled inside out into the catheter.

Such a catheter is awkward to use and not suitable for use in the gastrointestinal system.

WO-A-93 21984, DE-A-2928635, WO-A-93 03777 and US-A-3894540 all relate to catheters for insertion in the respiratory tract. The catheters are provided with a flexible sheath to keep the catheter sterile before insertion. During insertion the sheath is withdrawn from the catheter and stays outside the body.

The present invention especially relates to catheters used to administer liquid food, medical preparations and the like in the gastrointestinal tract. For this purpose the catheter is carried with an end part through the oral cavity to the desired final position, for instance the stomach, via the oral cavity and the oesophagus. The oral cavity usually contains a large number of bacteria. Through insertion of a catheter these bacteria are carried into the stomach and into the vicinity of the wound of the patient. This can result in infections.

The invention has for its object to obviate this drawback. This is achieved according to the invention with an assembly according to claim 1. The catheter with sheath according to the invention is carried through the nasal-oral cavity and the oesophagus to a position at a distance from the final position. A part of the sheath then still protrudes from the mouth of the patient. The sheath is subsequently withdrawn, so that the catheter, which has remained sterile, is left behind. The bacteria possibly adhering to the outside of the sheath are therefore removed with the sheath.

In order to enhance insertion and to prevent damage to the gastrointestinal wall, the end part of the sheath remote from the part held in fixed position is preferably folded through about 45°. Sharp edges are hereby avoided. The two parts of the non-folded portion of the end part for insertion are mutually connected by means of spot welding. When the sheath is withdrawn this connection is broken by exerting a pulling force.

The sheath is preferably of a flexible plastic such that it encloses the catheter in close-fitting manner during insertion. In order to enhance release of the sheath, the inner layer of the sheath can be of a releasing material, for instance a covering layer of hydrophilic coating. Shortly before insertion of the catheter the sheath can be released from the catheter by admitting water through an aperture arranged in the sheath, so that the sheath can be removed in simple manner.

The invention will be further elucidated with reference to the drawing.

Fig. 1 shows the catheter according to the invention.

Fig. 2 shows the use of the catheter of fig. 1, and

Fig. 3-5 show another way of using the catheter according to the invention.

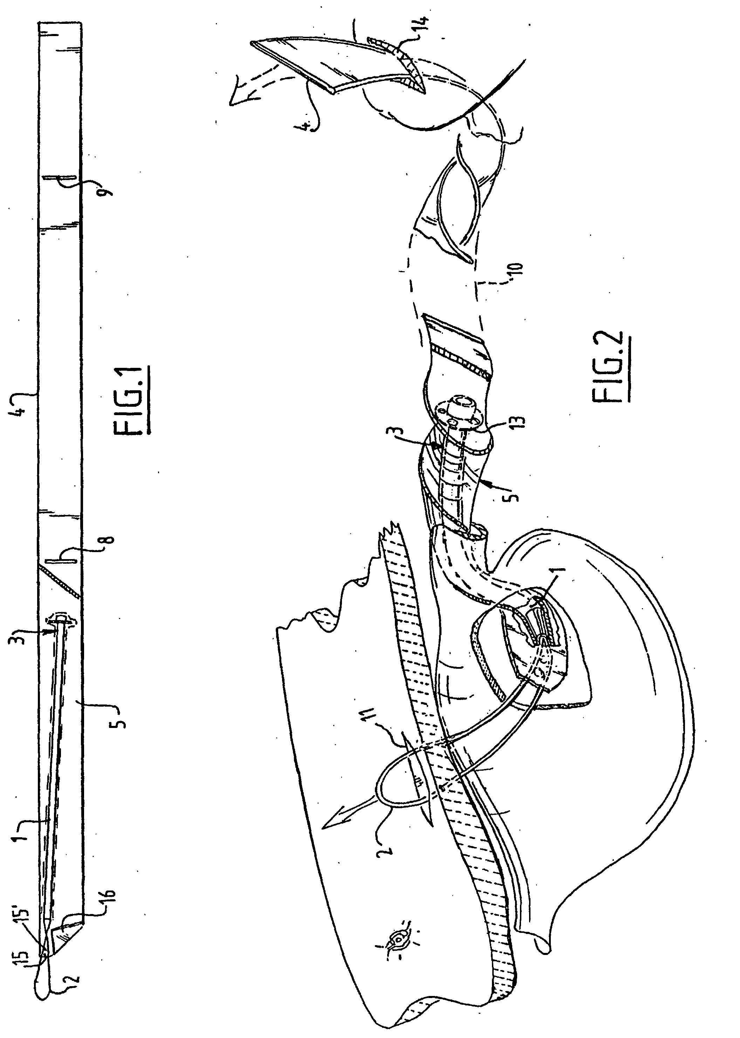

The catheter 1 according to fig. 1 as usual comprises on the one end a loop-shaped pulling thread 2 and on the other end part 3 a connection for a feed member, for instance for food. According to the invention the catheter 1 is received in a flexible sheath 4. The inside of sheath 4 is provided with a covering layer 5. Markings 8, 9 are arranged on the sheath in order to enable the passage of the sheath and the catheter into for instance the oesophagus to be followed. This can also take place by observing the markings on the part of the sheath still situated outside the body.

The catheter with sheath is carried via the oral cavity 14 into the oesophagus 10 and pulled out via an opening 11 arranged in the abdomen 12. On approaching a location to be kept sterile, the sheath 4 is withdrawn, wherein the spot welds 15,15' are broken. The catheter 1 therefore remains behind. The catheter is subsequently moved fully outward, wherein the element 13 of larger diameter ensures that the catheter remains fixed in the wall of the stomach.

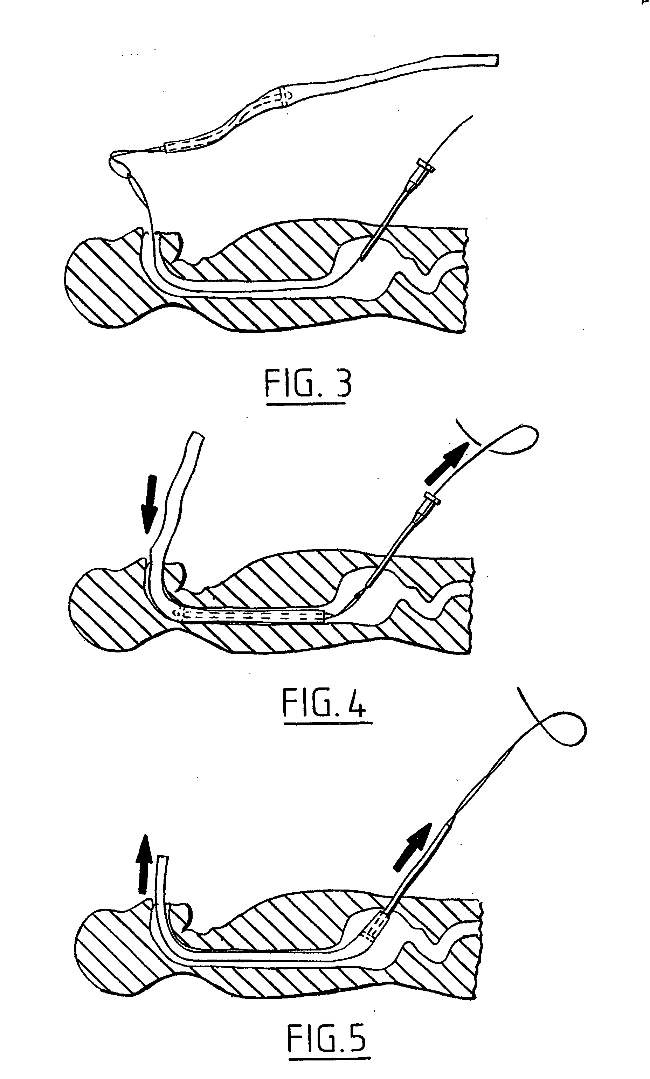

In the application outlined in fig. 3 to 5, the catheter with sheath is inserted via the oral cavity, wherein the sheath is withdrawn after a suitable position is reached.

## Claims

1. Assembly of a catheter (1) and a flexible sheath (4), **characterized in that** the assembly (1,4) is intended for insertion in the gastrointestinal system, the length of the sheath (4) is chosen greater than that of the catheter (1) and that the catheter (1) has a practically constant diameter and wall thickness over the entire length.

2. Assembly (1,4) as claimed in claim 1, **characterized in that** the sheath (4) is of flexible plastic.

3. Assembly (1,4) as claimed in claim 1 or 2, **characterized in that** the inner surface of the sheath (4) is provided with a coating layer (5).

4. Assembly (1,4) as claimed in claims 1-3, **characterized in that** the sheath (4) is provided with a bore.

5. Assembly (1,4) as claimed in claims 1-4, **characterized in that** the sheath (4) is folded over partially on the insertion end part.

6. Assembly (1,4) as claimed in claim 5, **characterized in that** the insertion end part is folded through about 45°.

7. Assembly (1,4) as claimed in claims 1-6, **characterized in that** the two parts of the non-folded portion of the insertion end part are mutually connected by means of spot welds (15,15').

## Patentansprüche

1. Anordnung aus einem Katheter (1) und einer flexiblen Hülle (4),
**dadurch gekennzeichnet, dass** die Anordnung (1, 4) zur Einführung in das Gastrointestinalsystem bestimmt ist, dass die Länge der Hülle (4) größer als die des Katheters (1) gewählt ist und dass der Katheter (1) über seine gesamte Länge praktisch konstanten Durchmesser und Wanddicke aufweist.

2. Anordnung (1, 4) nach Anspruch 1,
**dadurch gekennzeichnet, dass** die Hülle (4) aus flexiblem Kunststoff ist.

3. Anordnung (1, 4) nach Anspruch 1 oder 2,
**dadurch gekennzeichnet, dass** die Innenfläche der Hülle (4) mit einer Beschichtung (5) versehen ist.

4. Anordnung (1, 4) nach Anspruch 1 bis 3,
**dadurch gekennzeichnet, dass** die Hülle (4) mit einer Bohrung versehen ist.

5. Anordnung (1, 4) nach Anspruch 1 bis 4,
**dadurch gekennzeichnet, dass** die Hülle (4) am Einführungsende teilweise umgefaltet ist.

6. Anordnung (1, 4) nach Anspruch 5,
**dadurch gekennzeichnet, dass** das Einführungsende um etwa 45° gefaltet ist.

7. Anordnung (1, 4) nach den Ansprüchen 1 bis 6,
**dadurch gekennzeichnet, dass** die beiden Teile des nichtgefalteten Abschnitts des Einführungsendes durch Punktschweißungen (15, 15') miteinander verbunden sind.

## Revendications

1. Assemblage d'un cathéter (1) et d'une gaine flexible (4), **caractérisé en ce que** l'assemblage (1, 4) est destiné à être introduit dans le système gastro-intestinal, la longueur de la gaine (4) est choisie de manière à être supérieure à celle du cathéter (1), et le cathéter a un diamètre et une épaisseur de la paroi pratiquement constants sur toute la longueur.

2. Assemblage (1, 4) selon la revendication 1, **caractérisé en ce que** la gaine (4) est en plastique flexible.

3. Assemblage (1, 4) selon la revendication 1 ou 2, **caractérisé en ce que** la surface intérieure de la gaine (4) est fournie avec une couche de revêtement (5).

4. Assemblage (1, 4) selon les revendications 1 à 3, **caractérisé en ce que** la gaine (4) est fournie avec un orifice de passage.

5. Assemblage (1, 4) selon les revendications 1 à 4, **caractérisé en ce que** la gaine (4) est partiellement repliée sur l'extrémité d'introduction.

6. Assemblage (1, 4) selon la revendication 5, **caractérisé en ce que** l'extrémité d'introduction est pliée jusqu'à un angle d'environ 45°.

7. Assemblage (1, 4) selon les revendications 1 à 6, **caractérisé en ce que** les deux parties de la partie non pliée de l'extrémité d'introduction sont raccordées l'une à l'autre au moyen de points de soudure (15, 15').
